# EUROPÄISCHE PATENTSCHRIFT

(11) **EP 3 554 691 B1**
(45) Veröffentlichungstag und Bekanntmachung des Hinweises auf die Patenterteilung: **04.03.2026**
(21) Anmeldenummer: 17811211.6
(22) Anmeldetag: 17.11.2017
(51) Int. Cl.: B01J 23/889, B01J 37/10, B01J 37/03, C07C 29/154, B01J 37/00, B01J 35/00, B01J 23/00, B01J 37/18, B01J 37/08, B01J 35/37, B01J 35/40, B01J 35/50

(54) **TABLETTIERTER KUPFER-MANGAN-BASIERTER KATALYSATOR MIT ERHÖHTER STABILITÄT GEGENÜBER SÄUREEINWIRKUNG**
PELLETISED COPPER-MANGANESE-BASED CATALYST WITH INCREASED RESISTANCE TO ACIDIC ENVIRONMENT
CATALYSEUR EN PASTILLE À BASE DE CUIVRE ET MANGANÈSE AVEC UNE RÉSISTANCE AMÉLIORÉE À L'ENVIRONNEMENT ACIDE

(30) Priorität: 15.12.2016 DE 102016225172
(43) Veröffentlichungstag der Anmeldung: 23.10.2019
(73) Patentinhaber: Clariant International Ltd, 4132 Muttenz (CH)
(72) Erfinder: PFANZELT, Manuel, 82054 Sauerlach (DE); PAULUS, Martin, 83024 Rosenheim (DE); GROSSMANN, Frank, 81667 Muenchen (DE)
(74) Vertreter: Zusammenschluss Clariant
(86) Internationale Anmeldenummer: PCT/EP2017/079612
(87) Internationale Veröffentlichungsnummer: WO 2018/108451

(56) Entgegenhaltungen:
- EP-A1- 0 922 688
- EP-A2- 0 528 305
- WO-A1-2016/154514
- DE-A1- 102005 032 726
- US-A- 6 020 285

## Beschreibung

Die vorliegende Erfindung betrifft einen verbesserten Katalysator auf Basis eines tablettierten Katalysatorformkörpers umfassend ein Material der Formel CuAlₐMn_{b}Zn_{c}O_{d} mit Calciumaluminat als Bindermaterial zur Hydrierung von Carbonylgruppen in organischen Verbindungen, dadurch gekennzeichnet, dass der Katalysatorformkörper einen Gewichtsanteil von Calciumaluminat in einer Menge von 0,5 bis 10,0 Gew.-%, bezogen auf den Katalysatorformkörper, umfasst und die Seitendruckfestigkeit 100-300 N beträgt, bestimmt gemäß DIN EN 1094-5. Die vorliegende Erfindung betrifft außerdem auch die Herstellung des Katalysators sowie dessen Verwendung in der Hydrierung von Carbonylgruppen in organischen Verbindungen.

### Hintergrund der Erfindung

Katalytische Verfahren zur Hydrierung von Carbonylgruppen in organischen Verbindungen wie Estern, Diestern, Aldehyden oder Ketonen haben in der Industrie große Relevanz. Sie dienen unter anderem dazu, Carbonsäuren oder deren Ester, speziell Ester der Fettsäuren in die entsprechenden Alkohole umzuwandeln.

Als Katalysatoren eignen sich dabei Systeme auf Basis von Kupfer in Kombination mit weiteren Elementen der Übergangsmetalle. Die Katalysatoren liegen üblicherweise in Form von Tabletten, Extrudaten oder Granulaten vor.

Die WO 2004/085356 beschreibt die Herstellung eines Katalysators für die Hydrierung von Carbonylverbindungen, der neben Kupfer und Aluminium mindestens ein Oxid des Lanthan, Wolfram, Molybdän, Titan oder Zirkonium enthält, und dem weiterhin Kupferpulver oder - blättchen, Zementpulver oder Graphit beigemengt ist.

In der US 6,020,285 wird die Herstellung eines Cobalt oder Nickel enthaltenden Katalysators beschrieben, der außerdem Calciumaluminat mit einem Al/Ca-Verhältnis von größer 2,5 umfasst. Der Katalysator eignet sich zur Zersetzung von Hypochlorit.

Die WO 98/11985 offenbart Cobalt oder Nickel enthaltende Katalysatoren, die darüber hinaus noch Calciumaluminat mit einem Al/Ca-Verhältnis von größer 4,0 sowie Alumina und/oder Magnesia umfasst. Der Katalysator eignet sich zur Zersetzung oxidierender Substanzen.

In der US 7,084,312 wird die Herstellung von Katalysatoren auf Basis von Kupfer, Zink und Aluminium beschrieben, für die ein oxidisches Gemisch von Kupfer Zink und Aluminium mit metallischem Kupfer, einem Zement oder einer Mischung beider Materialien vermengt und zu Tabletten geformt wird. Der Katalysator wird für die Hydrierung von organischen Verbindungen, die Carbonylgruppen aufweisen, verwendet.

Yakerson et al. (Scientific Bases for the Preparation of Heterogeneous Catalysts, Preparation of Catalysts, S. 879 ff.) beschreibt die Herstellung von zementhaltigen Metallkatalysatoren, wie z.B. Nickel-, Kupfer- oder Zinkhaltigen Katalysatoren. Hierfür werden die entsprechenden Metallhydroxocarbonate verwendet.

DE 10 2005 032726 A1 offenbart einen Katalysator sowie ein Verfahren zur Hydrierung einer mindestens eine Carbonylgruppe aufweisenden organischen Verbindung.

Die Ausgangsverbindungen der Hydrierungsprozesse weisen in der Regel Spuren von sauren Verbindungen auf. Bei diesen handelt es sich beispielsweise um Carbonsäuren, die als Nebenprodukte in Veresterungsreaktionen vorliegen. Diese Verbindungen greifen unter den Reaktionsbedingungen der Hydrierungsreaktion den Katalysator an und führen zu einer Erniedrigung der mechanischen Stabilität und dem teilweise zu beobachtenden Auswaschen der katalytisch aktiven Metalle, die mit dem Produktstrom aus dem Reaktionsreaktor herausgetragen werden und von ihm abgetrennt werden müssen. Zudem reduziert sich mit fortschreitendem Austrag der katalytisch aktiven Metalle auch die katalytische Aktivität des Katalysators.

Für solche Reaktionen werden Katalysatoren verwendet, die kupfer- und chromhaltig sind.

Diese weisen üblicherweise eine erhöhte Stabilität gegenüber Säureeinwirkung auf. Aufgrund von strengeren Umweltauflagen ist der Einsatz von chromhaltigen Katalysatoren mit immer höheren Anforderungen verbunden, so dass der Bedarf besteht, die bestehenden CuCr-Systeme durch umweltverträgliche Alternativen zu ersetzen, die dennoch vergleichbare katalytische und physikalische Eigenschaften aufweisen.

Die Aufgabe der vorliegenden Erfindung bestand deshalb darin, einen Katalysator für Hydrierungen von Carbonylgruppen in organischen Verbindungen bereitzustellen, der sich durch eine verbesserte mechanische Stabilität auszeichnet und der weniger anfällig gegenüber der Einwirkung von sauren Verbindungen ist.

Diese Aufgabe wird durch den erfindungsgemäßen Katalysator gelöst.

### Beschreibung der Erfindung

Die Erfindung betrifft einen Katalysatorformkörper in Tablettenform, umfassend ein Material der Formel CuAlₐMn_{b}Zn_{c}O_{d} und Calciumaluminat als Bindermaterial in einem Gewichtsanteil von 0,5 bis 10,0 %, bezogen auf den Katalysatorformkörper, wobei die Seitendruckfestigkeit 100-300 N beträgt, bestimmt gemäß DIN EN 1094-5. a beträgt zwischen 0 und 2,5, b zwischen 0,001 und 0,6, c zwischen 0 und 2,5, und d ist so gewählt, dass die Gesamtladung der Summenformel null ist. In einer besonderen Ausführungsform beträgt a zwischen 0,8 und 1,2, b zwischen 0,05 und 0,3, c 0 und d ist so gewählt, dass die Gesamtladung der Summenformel null ist. In einer weiteren bevorzugten Ausführungsform hat a den Wert 0, b beträgt zwischen 0,04 und 0,1, c zwischen 0,8 und 1,5 und d ist so gewählt, dass die Gesamtladung der Summenformel null ist.

Die Oxidationsstufen der Elemente betragen +2 für Cu, +3 für Al und +2 für Zn. Je nach Mengenanteil des Mn beträgt die Oxidationsstufe für Mn +2, +3 oder +4, wobei auch Materialien vorliegen können, in denen einem Teil der Mn-Atome die Oxidationsstufe +2 und anderen die Oxidationsstufe +3 oder +4 zugeordnet ist, so dass eine gemittelte Oxidationsstufe im Bereich von +2 bis +4 resultiert.

Bei dem Calciumaluminat handelt es sich um eine Verbindung, die Ca und Al in Form von Oxiden und/oder Hydroxiden enthält. Beispielsweise handelt es sich um gebrannte Calciumaluminate der allgemeinen Formel x CaO · y Al2O3 oder um chemisch gefällte Calciumaluminate der allgemeinen Formel CaₓAl_{y}(OH)_{z}. Abhängig von der Behandlung der Calciumaluminate können aber auch Zwischenstufen dieser beiden Summenformeln vorliegen, die ebenfalls als Bindermaterial geeignet sind. Neben diesen Elementen können im Calciumaluminat noch weitere Elemente vorliegen. In einer bevorzugten Ausführungsform enthält das Calciumaluminat weitere Elemente in einem Gewichtsanteil von kleiner 5,0 Gew.-%, bevorzugt kleiner 1,0 Gew.-% und besonders bevorzugt kleiner 0,1 Gew.-%, bezogen auf das Gewicht des Calciumaluminats.

Der erfindungsgemäße Katalysatorformkörper ist dadurch gekennzeichnet, dass er Calciumaluminat als Bindermaterial in einem Gewichtsanteil von 0,5 bis 10,0 %, bezogen auf den Katalysatorformkörper, enthält. Bevorzugt beträgt der Anteil von 0,5 bis 5,0 % besonders bevorzugt von 0,5 % bis kleiner 5,0 %, am bevorzugtesten 0,5 bis 3,0 %, bezogen auf den Katalysatorformkörper.

Das atomare Ca/Al-Verhältnis des Calciumaluminats, das in der vorliegenden Erfindung eingesetzt wird, kann variieren und beträgt bevorzugt zwischen 0,9 und 3,5, noch mehr bevorzugt zwischen 1,0 und 2,0.

Als Calciumaluminate eignen sich synthetisch hergestellte Materialien. Es können aber auch natürlich vorkommende Calciumaluminate verwendet werden, wie z.B. Katoit.

Der tablettierte Katalysatorformkörper kann in verschiedenen Abmessungen vorliegen. Der Durchmesser der Tabletten kann dabei zwischen 2 und 6 mm und bevorzugt zwischen 2 und 4 mm liegen. Besonders bevorzugt beträgt der Durchmesser 3 mm. Die Höhe der Tabletten kann zwischen 2 und 6 mm und bevorzugt zwischen 2 und 4 mm liegen. Besonders bevorzugt beträgt die Höhe 3mm.

Das Calciumaluminat kann vor der Verwendung als Bindermaterial einer thermischen Behandlung (Kalzinierung) unterzogen werden. Diese findet bei einer Temperatur zwischen 300 und 800 °C, bevorzugt zwischen 450 und 750 °C und besonders bevorzugt zwischen 450 und 650 °C statt.

In einer Ausführungsform der Erfindung weisen die Partikel des Calciumaluminats eine durchschnittliche Partikelgröße mit einem d₅₀-Wert im Bereich von 0,1 bis 200 µm, bevorzugt im Bereich von 5 bis 50 µm , gemessen mittels Laser Sizing nach ISO 13302/2009, auf. In einer weiteren Ausführungsform liegt der d₉₀-Wert liegt im Bereich von 10 bis 300 µm, bevorzugt im Bereich von 20 bis 100 µm.

Der erfindungsgemäße Katalysatorformkörper umfassend ein Material der Formel CuAlₐMn_{b}Zn_{c}O_{d} und enthaltend Calciumaluminat als Bindermaterial in einem Gewichtsanteil von 0,5 bis 10,0 %, bezogen auf den Katalysatorformkörper, wird hergestellt durch folgende erfindungsgemäße Schritte:
a) Mischen eines metallhaltigen Gemischs, enthaltend Kupfer, Mangan und mindestens ein Element, ausgewählt aus Zink und Aluminium, mit Calciumaluminat, einem Schmiermittel und Wasser,
b) Tablettierung der Mischung nach Schritt a) zum Erhalt eines tablettierten Formkörpers,
c) Thermische Behandlung der tablettierten Formkörper bei einer Temperatur zwischen 200 und 800 °C für eine Dauer zwischen 30 min und 4 h,
   wobei a zwischen 0 und 2,5, b zwischen 0,001 und 0,6 und c zwischen 0 und 2,5 beträgt, und d so gewählt ist, dass die Gesamtladung der Summenformel null ist.

In einer besonderen Ausführungsform beträgt a zwischen 0,8 und 1,2, b zwischen 0,05 und 0,3, c 0 und d ist so gewählt, dass die Gesamtladung der Summenformel null ist. In einer weiteren bevorzugten Ausführungsform hat a den Wert 0, b beträgt zwischen 0,04 und 0,1, c zwischen 0,8 und 1,5 und d ist so gewählt, dass die Gesamtladung der Summenformel null ist.

In einer bevorzugten Ausführungsform beträgt der Gewichtsanteil des Calciumaluminats von 0,5 bis 5,0 % bevorzugter von 0,5 % bis kleiner 5,0 %, am bevorzugtesten 0,5 bis 3,0 %, bezogen auf den Katalysatorformkörper.

Das in Schritt a) eingesetzte Gemisch enthaltend Kupfer, Mangan und mindestens ein Element, ausgewählt aus Zink und Aluminium, kann aus der Gruppe der Oxide, Hydroxide oder Carbonate gewählt werden. Als bevorzugt eignen sich hierbei die Oxide der entsprechenden Elemente. Die Elemente können dabei sowohl als einzelne Verbindungen, wie Kupferoxid und Oxid des Mangan, Zink oder Aluminium, vorliegen als auch als Mischverbindungen wie Mischoxide des Kupfers, des Mangans und mindestens einem Element, ausgewählt aus Zink und Aluminium.

Das metallhaltige Gemisch aus Schritt a) kann durch die Fällung der gelösten Metallionen aus wässriger Lösung erhalten werden. Als Ausgangsverbindungen eignen sich dabei prinzipiell alle Verbindungen, die in Wasser, basischen oder sauren wässrigen Lösungen löslich sind. Bevorzugt werden Nitrate, Halogenide, Oxide, Sulfate, Acetate oder Formiate eingesetzt.

Die nach Schritt a) erhaltene Mischung kann anschließend optional einem Alterungsschritt unterzogen werden. Dabei wird die Mischung für 5 min bis 10 h, bevorzugt für 5 min bis 3 h stehengelassen, ohne dass weitere Komponenten zugegeben werden oder die Mischung in Bewegung gehalten wird. Die Alterungstemperatur entspricht üblicherweise der Umgebungstemperatur der Mischung, sie kann aber innerhalb eines Bereichs von 0 °C bis 90 °C kontrolliert eingestellt werden.

Die nach Schritt a) erhaltene Mischung, die gegebenenfalls noch gealtert wurde, wird anschließend üblicherweise ohne eine thermische Behandlung gegebenenfalls kompaktiert und/oder granuliert und dann einem Tablettierschritt b) unterzogen. Dabei werden handelsübliche Tablettiermaschinen eingesetzt, beispielsweise vom Typ Pressima der Firma IMA Kilian. Die Mischung nach Schritt a) enthält ein Schmiermittel. Bei diesem handelt es sich um eine Verbindung, die die Tablettiereigenschaften des Gemischs unterstützt. Geeignete Schmiermittel sind Graphit, Öle oder Stearate, bevorzugt Graphit. Das Schmiermittel wird der zu tablettierenden Masse in einem Anteil von 0,1 bis 5,0 Gew.-% zugegeben, bevorzugt von 0,5 bis 5,0 Gew.-% und besonders bevorzugt von 1,0 bis 4,0 Gew.-%.

Die Mischung nach Schritt a) enthält außerdem Wasser. Dieses liegt üblicherweise in einer Menge von 1 bis 10 Gew.-%, bezogen auf das eingesetzte metallhaltige Gemisch, vor, bevorzugt von 2 bis 4 Gew.-%, ganz besonders von 3 Gew.-%.

Die thermische Behandlung der Tabletten erfolgt bei einer Temperatur zwischen 200 und 800 °C, bevorzugt zwischen 300 und 700 °C, bevorzugter zwischen 300 und 500 °C. Die Dauer dieser thermischen Behandlung beträgt zwischen 30 min und 4 h, bevorzugt zwischen 1 und 3 h und besonders bevorzugt 2 h.

Die mit dem erfindungsgemäßen Verfahren hergestellten Tabletten weisen eine Seitendruckfestigkeit von 100 bis 300 N, bevorzugt 100 bis 250 N, besonders bevorzugt 120 bis 180 N auf. Vorzugsweise weisen die durch das Tablettieren hergestellten Tabletten einen Durchmesser im Bereich von 2 bis 4 mm, eine Höhe im Bereich von 2 bis 4 mm und eine Seitendruckfestigkeit im Bereich von 120 bis 180 N auf.

Das Porenvolumen (gemessen mittels Quecksilberporosimetrie) der erfindungsgemäßen Katalysatorformkörpers beträgt zwischen 100 und 300 mm³/g, vorzugsweise zwischen 150 und 250 mm³/g.

Die erfindungsgemäßen Katalysatorformkörper weisen eine spezifische BET-Oberfläche von 20 bis 60 m²/g, bevorzugt von 30 bis 50 m²/g auf.

Vorzugsweise beträgt der Anteil des Porenvolumens der Poren mit einem Radius von 7,0 bis 40,0 nm des erfindungsgemäßen Katalysatorformkörpers zwischen 50 und 95 %, bevorzugt zwischen 70 und 90 % des Gesamtporenvolumens.

Der durch das erfindungsgemäße Verfahren erhältliche Katalysatorformkörper wird in einem weiteren Schritt reduziert.

Die Reduzierung erfolgt dabei bevorzugt durch Erhitzen des tablettierten Katalysatorformkörpers in einer reduzierenden Atmosphäre. Bei der reduzierenden Atmosphäre handelt es sich insbesondere um Wasserstoff. Die Reduzierung erfolgt beispielsweise bei einer Temperatur im Bereich von 150°C bis 450°C, bevorzugt im Bereich von 180°C bis 250°C, besonders bevorzugt im Bereich von 190°C bis 210°C. Die Reduzierung erfolgt beispielsweise über einen Zeitraum von 1 Stunde bis 10 Tage, bevorzugt über einen Zeitraum von 2 Stunden bis 72 Stunden, besonders bevorzugt über einen Zeitraum von 24 bis 48 Stunden. In einer bevorzugten Ausführungsform erfolgt das Reduzieren bei einer Temperatur im Bereich von 190°C bis 210°C über einen Zeitraum von 24 bis 48 Stunden.

In einer bevorzugten Ausführungsform werden die Katalysatorformkörper nach der Reduktion nass oder trocken stabilisiert. Bei der Nassstabilisierung werden die Katalysatorformkörper mit einer Flüssigkeit überschichtet, um den Kontakt mit Sauerstoff möglichst zu vermeiden. Geeignete Flüssigkeiten umfassen organische Flüssigkeiten und Wasser, bevorzugt organische Flüssigkeiten. Bevorzugte organische Flüssigkeiten sind solche, die bei 20°C einen Dampfdruck von 0,5 hPa oder weniger aufweisen. Beispiele solcher geeigneter organischer Flüssigkeiten sind Iso-Decanol, Nafol, Fettalkohole, Hexadecan, 2-Ethyl-hexanol, Propylenglycol und Mischungen davon, besonders Iso-Decanol. Bei der Trockenstabilisierung wird in den Reduktionsraum ein Gemisch aus Sauerstoff oder einem sauerstoffhaltigen Gas, bevorzugt Luft, und einem inerten Gas, wie Argon oder Stickstoff zudosiert. Die Konzentration an Sauerstoff in dem Gemisch wird vorzugsweise von etwa 0,04 Vol.-% auf etwa 21 Vol.-% erhöht. Beispielsweise kann ein Gemisch aus Luft und Inertgas zudosiert werden, wobei das Verhältnis von Luft zu Inertgas anfangs etwa 0,2 Vol.-% Luft zu 99,8 Vol.-% Inertgas beträgt. Das Verhältnis von Luft zu Inertgas wird dann allmählich erhöht (z.B. kontinuierlich oder schrittweise), bis schließlich beispielsweise 100 Vol.-% Luft zudosiert wird (entsprechend einer Sauerstoffkonzentration von etwa 21 Vol.-%). Ohne an eine Theorie gebunden zu sein, wird vermutet, dass durch die Zudosierung von Luft oder Sauerstoff eine dünne Oxidschicht mit einer Dicke von beispielsweise 0,5 bis 50 nm, bevorzugt 1 bis 20 nm, besonders bevorzugt 1 bis 10 nm an der Oberfläche des Katalysators entsteht, welche den Katalysatorformkörper vor weiterer Oxidation schützt. Bei der Trockenstabilisierung beträgt die Reaktortemperatur bevorzugt 100°C oder weniger, besonders bevorzugt 20°C bis 70°C und am meisten bevorzugt 30°C bis 50°C. Die Reduzierung kann ex situ oder in situ in der Reaktionsanlage erfolgen, in der der Katalysatorformkörper als Katalysator eingefüllt wird.

Die Seitendruckfestigkeit der Katalysatorformkörper in Tablettenform weisen nach der Reduzierung Werte von 50 bis 250 N, bevorzugt 60 bis 200 N, besonders bevorzugt 70 bis 150 N auf.

Zur Bestimmung der Stabilität der erfindungsgemäßen Katalysatorformkörper gegenüber Säureeinwirkung wird der Formkörper einer Säurebehandlung unterzogen und anschließend die Seitendruckfestigkeit der so behandelten Tabletten bestimmt.

Der erfindungsgemäße Katalysatorformkörper ist für die Verwendung in katalytischen Hydrierungen von Carbonylgruppen in organischen Verbindungen geeignet. Mögliche Reaktionen umfassen die Hydrierung von Diestern (insbesondere von Maleinsäurediestern) zu Diolen, Hydrierung von Zuckern zu Polyolen, Hydrierung von Estern, insbesondere von Fettsäureestern, Hydrierung einer Fettsäure (z.B. durch Veresterung und anschließender Hydrogenolyse) Hydrierung eines Ketons, Hydrierung von Oxoaldehyden zu Oxoalkoholen und die Hydrierung von Furfural.

### Beispiele

Die Bestimmungen des Glühverlusts im Rahmen der vorliegenden Erfindung erfolgten, indem von ca. 1-2 g einer Probe des zu analysierenden Materials deren Gewicht bestimmt wurde und diese anschließend unter Raumatmosphäre auf 900 °C aufgeheizt und bei dieser Temperatur für 3 h gelagert wurde. Anschließend wurde die Probe unter Schutzatmosphäre abgekühlt und das verbliebene Gewicht gemessen. Die Differenz aus Gewicht vor und nach der thermischen Behandlung entspricht dem Glühverlust.

Die Bestimmung der Seitendruckfestigkeit erfolgte gemäß DIN EN 1094-5. Hierbei wurde eine statistisch ausreichende Anzahl an Tabletten (mindestens 20 Tabletten) gemessen und der arithmetische Mittelwert der Einzelmessungen berechnet. Dieser Mittelwert entspricht der Seitendruckfestigkeit einer bestimmten Probe.

Die Bestimmung chemischer Elemente erfolgte mittels ICP-Messung (Inductively Coupled Plasma) nach DIN EN ISO 11885.

Die Säurebehandlung erfolgte, indem eine Gesamtmenge an tablettierten Proben von 1,5 g mit 15 g Essigsäure (10 Vol.-% in H₂O) vermengt wurde. Diese wurden für 30 min bei Raumtemperatur gerührt. Die tablettierte Probe wurde bei 120 °C für 10 h in Luft getrocknet und anschließend deren Seitendruckfestigkeit gemessen.

Die spezifischen BET-Oberflächen wurden mittels Stickstoffadsorption nach DIN 66131 bestimmt. Der durch das erfindungsgemäße Verfahren erhältliche Katalysator weist vorzugsweise eine BET-Oberfläche im Bereich von 20 bis 100 m²/g, insbesondere von 30 bis 80 m²/g und besonders bevorzugt von 40 bis 60 m²/g auf.

Das Porenvolumen des Katalysatorformkörpers wurde nach der Quecksilberporosimetrie-Methode gemäß DIN 66133 gemessen.

Der Gewichtsanteil des Calciumaluminats im Katalysatorformkörper wurde mittels Röntgendiffraktometrie ermittelt. Es wurde ein D4 Endeavor der Firma BRUKER verwendet. Dazu wurde die Probe über einen Bereich von 5 bis 90 2°*Θ* (Schrittfolge 0,020 2°*Θ*, 1,5 Sekunden Messzeit pro Schritt) gemessen. Als Strahlung wurde CuKα1-Strahlung (Wellenlänge 1,54060 Å, 40 kV, 35 mA) verwendet. Der Probenteller wurde während der Messung mit einer Geschwindigkeit von 30 Umdrehungen/min um seine Achse gedreht. Das erhaltene Spektrum der Reflexintensitäten wurde mittels Rietveld-Verfeinerung quantitativ analysiert und der Anteil von Calciumaluminat in der Probe bestimmt. Zur Bestimmung des Anteils der jeweiligen Kristallphasen wurde die Software TOPAS der Firma BRUKER verwendet.

### Herstellung des Katalysatorpulvers

Eine wässrige Lösung 1 wurde hergestellt, indem 1250 g Cu(NO₃)₂ · 3 H₂O, 220 g Mn(NO₃)₂ · 4 H₂O und 1800 g Al(NO₃)₃ · 9 H₂O in 9000 g destilliertem H₂O gelöst wurden. Lösung 2 wurde hergestellt, indem 1720 g Na₂CO₃ in 7500 g destilliertem H₂O gelöst wurden. Beide Lösungen wurden separat unter Rühren auf 80 °C aufgeheizt. Anschließend wurden beide Lösungen in einen Fällungsbehälter unter kontinuierlichem Rühren zu dosiert. Die Zugabe beider Lösungen erfolgte dabei so, dass die vereinte Mischung im Fällungsbehälter einen pH-Wert von 7 (+/-0,2) aufwies. Das hierbei ausgefällte Präzipitat wurde abfiltriert und mit destilliertem H₂O gewaschen, um anhaftende Verunreinigungen zu entfernen. Der Filterkuchen wurde in 8 L dest. H₂O resuspendiert und sprühgetrocknet. Das getrocknete Pulver wurde anschließend für 3 h bei 750 °C thermisch behandelt und diente als Ausgangsmaterial für die Tablettierungsbeispiele. Die relativen Gewichtsanteile betrugen Cu = 45 Gew. %, Mn = 7 Gew.-% und Al = 18 Gew.-%, bezogen auf die Gesamtmasse nach Glühverlust. Dies entspricht einer Summenformel von CuMn_{0,18}Al_{0,94}O2,6.

### Vergleichsbeispiel 1 (Katalysator A)

Katalysator A wurde hergestellt, indem 500 g des Katalysatorpulvers mit 10 g Graphit gemischt und anschließend zu Tabletten mit Abmessungen von 3 mm Höhe und 3 mm Durchmesser geformt wurden. Die Seitendruckfestigkeit der Probe wurde nach der Tablettierung bestimmt. Durch die Säurebehandlung wurden alle Tabletten so geschädigt, dass sie komplett in gebrochener Form vorlagen und eine Bestimmung der Seitendruckfestigkeit nicht mehr möglich war.

### Vergleichsbeispiel 2 (Katalysator B)

Katalysator B wurde hergestellt, indem 500 g des Katalysatorpulvers mit 10 g Graphit gemischt und anschließend zu Tabletten mit Abmessungen von 4,5 mm Höhe und 4,5 mm Durchmesser geformt wurden. Die Seitendruckfestigkeit der Probe wurde nach der Tablettierung und der Säurebehandlung bestimmt. Durch die Säurebehandlung wurden alle Tabletten so geschädigt, dass sie komplett in gebrochener Form vorlagen und eine Bestimmung der Seitendruckfestigkeit nicht mehr möglich war.

Ein Teil des nach der Tablettierung erhaltenen Materials wurde einer Reduzierung unterzogen. Dabei wurde die Probe in einem Gasgemisch aus 2 % Volumen-% H₂ und 98 Vol.-% N₂ bei einer Temperatur von 200 °C thermisch behandelt, um eine Reduzierung des vorliegenden CuO zu Cu zu bewirken. Anschließend wurde die Probe unter Stickstoff auf Raumtemperatur abgekühlt und unter flüssigem Decanol gelagert. Von dieser Probe wurde anschließend deren Seitendruckfestigkeit gemessen.

### Beispiel 1 (Katalysator 1)

500 g des Katalysatorpulvers wurden mit 5 g Calciumaluminat (Typ SECAR 71, 30 Gew.-% CaO, 70 Gew.-% Al₂O₃) , 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 320 °C. Die Seitendruckfestigkeit der Probe wurde nach der Tablettierung, nach der thermischen Behandlung sowie nach der Säurebehandlung bestimmt.

### Beispiel 2 (Katalysator 2)

500 g des Katalysatorpulvers wurden mit 15 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Die Tabletten wurden im Anschluss daran für 24 h bei 100 - 150 °C mit Wasserdampf behandelt. Anschließend erfolgte eine thermische Behandlung bei 320°C. Die Seitendruckfestigkeit der Probe wurde nach der Tablettierung, nach der thermischen Behandlung sowie nach der Säurebehandlung der kalzinierten Probe bestimmt.

### Beispiel 3 (Katalysator 3)

500 g des Katalysatorpulvers wurden mit 15 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 4,5 mm Höhe und 4,5 mm Durchmesser geformt. Anschließend erfolgte eine thermische Behandlung bei 450 °C. Die Seitendruckfestigkeit der Probe wurde nach der Tablettierung, nach der thermischen Behandlung sowie nach der Säurebehandlung der kalzinierten Probe bestimmt.

Ein Teil des nach der Tablettierung erhaltenen Materials wurde einer Reduzierung unterzogen. Dabei wurde die Probe in einem Gasgemisch aus 2 % Volumen-% H₂ und 98 Vol.-% N₂ bei einer Temperatur von 200 °C thermisch behandelt, um eine Reduzierung des vorliegenden CuO zu Cu zu bewirken. Anschließend wurde die Probe unter Stickstoff auf Raumtemperatur abgekühlt und unter flüssigem Decanol gelagert. Von dieser Probe wurde anschließend deren Seitendruckfestigkeit gemessen.

### Beispiel 4 (Katalysator 4)

500 g des Katalysatorpulvers wurden mit 15 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 450 °C. Die Seitendruckfestigkeit der Probe wurde nach der Tablettierung, nach der thermischen Behandlung sowie nach der Säurebehandlung der kalzinierten Probe bestimmt.

### Beispiel 5 ( Katalysator 5)

500 g des Katalysatorpulvers wurden mit 15 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 650 °C. Die Seitendruckfestigkeit der Probe wurde nach der Tablettierung, nach der thermischen Behandlung sowie nach der Säurebehandlung der kalzinierten Probe bestimmt.

### Beispiel 6 (Katalysator 6)

500 g des Katalysatorpulvers wurden mit 50 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 450 °C. Die Seitendruckfestigkeit der Probe wurde nach der Tablettierung, nach der thermischen Behandlung sowie nach der Säurebehandlung der kalzinierten Probe bestimmt.

### Beispiel 7 (Katalysator 7)

500 g des Katalysatorpulvers wurden mit 50 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 650 °C. Die Seitendruckfestigkeit der Probe wurde nach der Tablettierung, nach der thermischen Behandlung sowie nach der Säurebehandlung der kalzinierten Probe bestimmt.

### Beispiel 8 (Katalysator 8) (nicht erfindungsgemäß)

500 g des Katalysatorpulvers wurden mit 100 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 450 °C. Die Seitendruckfestigkeit der Probe wurde nach der Tablettierung, nach der thermischen Behandlung sowie nach der Säurebehandlung der kalzinierten Probe bestimmt.

### Beispiel 9 (Katalysator 9) (nicht erfindungsgemäß)

500 g des Katalysatorpulvers wurden mit 100 g Calciumaluminat (Typ SECAR 71, 30 Gew.-% CaO, 70 Gew.-% Al₂O₃), 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 650 °C. Die Seitendruckfestigkeit der Probe wurde nach der Tablettierung, nach der thermischen Behandlung sowie nach der Säurebehandlung der kalzinierten Probe bestimmt.

### Beispiel 10 (Katalysator 10)

Das in Beispiel 1 verwendete Calciumaluminat wurde bei 650 °C für 2 h thermisch behandelt. Anschließend wurden 500 g des Katalysatorpulvers mit 15 g dieses thermisch behandelten Calciumaluminats, 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 450 °C. Die Seitendruckfestigkeit der Probe wurde nach der Tablettierung, nach der thermischen Behandlung sowie nach der Säurebehandlung der kalzinierten Probe bestimmt.

### Beispiel 11 (Katalysator 11)

Das in Beispiel 1 verwendete Calciumaluminat wurde bei 650 °C für 2 h thermisch behandelt. Anschließend wurden 500 g des Katalysatorpulvers mit 15 g dieses thermisch behandelten Calciumaluminats, 10 g Graphit und 15 g destilliertem H₂0 gemischt. Anschließend wurde das Gemisch für 4 h gealtert und zu Tabletten mit Abmessungen von 3 mm Höhe und 3 mm Durchmesser geformt. Anschließend erfolgte eine thermische Behandlung bei 650 °C. Die Seitendruckfestigkeit der Probe wurde nach der Tablettierung, nach der thermischen Behandlung sowie nach der Säurebehandlung der kalzinierten Probe bestimmt.

| Beispiel | Calciumaluminat [Gew.-%] | Temperatur der thermischen Behandlung [°C] | Seitendruckfestigkeit nach Tablettierung [N] | Seitendruckfestigkeit nach thermischer Behandlung [N] | Seitendruckfestigkeit nach Säurebehandlung [N] | Seitendruckfestigkeit nach Reduzierung [N] | Abmessungen [mm x mm] | Rel. Porenvolumen der Poren im Bereich von Radien von 40 - 7,0 nm [%] | BET-Oberfläche [m²/g] |
|---|---|---|---|---|---|---|---|---|---|
| Katalysator A | - | keine thermische Behandlung | 85 | - | 0 | 0 | 3 x 3 | 69,6 | 57 |
| Katalysator B | - | Keine thermische Behandlung | 99 | - | 0 | 69 | 4,5 x 4,5 | 87,3 | 57 |
| Katalysator 1 | 1 | 320 | 64 | 151 | 129 | nicht bestimmt | 3 x 3 | 71,7 | 53 |
| Katalysator 2 | 3 | 320 | 73 | 162 | 109 | nicht bestimmt | 3 x 3 | 74,8 | 53 |
| Katalysator 3 | 3 | 450 | 82 | 230 | 190 | 134 | 4,5 x 4,5 | 83,1 | 39 |
| Katalysator 4 | 3 | 450 | 88 | 175 | 122 | nicht bestimmt | 3 x 3 | 87,3 | 41 |

| | | | | | | | | | |
|---|---|---|---|---|---|---|---|---|---|
| Katalysator 5 | 3 | 650 | 88 | 233 | 170 | nicht bestimmt | 3 x 3 | 87,5 | 35 |
| Katalysator 6 | 10 | 450 | 66 | 134 | 93 | nicht bestimmt | 3 x 3 | 84,7 | 44 |
| Katalysator 7 | 10 | 650 | 70 | 175 | 141 | nicht bestimmt | 3 x 3 | 87,1 | 36 |
| Katalysator 8 | 20 | 450 | 70 | 122 | 90 | nicht bestimmt | 3 x 3 | 82,0 | 39 |
| Katalysator 9 | 20 | 650 | 71 | 161 | 140 | nicht bestimmt | 3 x 3 | 78,0 | 35 |
| Katalysator 10 | 3 | 450 | 90 | 216 | 145 | nicht bestimmt | 3 x 3 | 89,5 | 40 |
| Katalysator 11 | 3 | 450 | 104 | 253 | 165 | nicht bestimmt | 3 x 3 | 86,3 | 40 |

Anhand von Tabelle 1 erkennt man deutlich, dass die erfindungsgemäßen Katalysatoren nach der thermischen Behandlung eine deutlich höhere Seitendruckfestigkeit als der Vergleichskatalysator aufweisen. Nach der Säurebehandlung nimmt die Seitendruckfestigkeit für alle Proben ab, wobei die Seitendruckfestigkeit der erfindungsgemäßen Proben weiterhin deutlich höher ist als die der Vergleichskatalysatoren Katalysator A und B vor der Säurebehandlung. Dies unterstreicht die erhöhte mechanische Stabilität der erfindungsgemäßen Katalysatoren auch unter den drastischen Bedingungen einer säurehaltigen Umgebung, wie sie auch bei einer Hydrierung von Carbonylgruppen in organischen Verbindungen wie Estern, Diestern, Aldehyden oder Ketonen vorliegt, da die Edukte säurehaltige Verunreinigungen (enthalten.

Die spezifischen Oberflächen nach BET werden durch das erfindungsgemäße Verfahren nicht wesentlich beeinflusst. Gleiches gilt auch für das relative Porenvolumen der Poren im Bereich von 40 - 7, 0 nm. Dies unterstreicht die Stabilität der erfindungsgemäßen Katalysatorformkörper gegenüber der thermischen Belastung während der thermischen Behandlung.

## Patentansprüche

1. Katalysatorformkörper umfassend ein Material der Formel CuAlₐMn_{b}Zn_{c}O_{d}, wobei a für eine Zahl zwischen 0 und 2,5 steht, b für eine Zahl zwischen 0,001 und 0,6 und c für eine Zahl zwischen 0 und 2,5, und d so gewählt ist, dass die Gesamtladung der Summenformel null ist, **dadurch gekennzeichnet, dass** der Katalysatorformkörper in Tablettenform vorliegt und Calciumaluminat als Bindermaterial in einem Gewichtsanteil von 0,5 bis 10,0 %, bezogen auf den Katalysatorformkörper, enthält und die Seitendruckfestigkeit 100-300 N beträgt, bestimmt gemäß DIN EN 1094-5.

2. Katalysatorformkörper nach Anspruch 1, wobei das Calciumaluminat in einem Gewichtsanteil 0,5 bis 5,0 %, bevorzugt von 0,5 % bis kleiner 5,0 %, bevorzugter von 0,5 bis 3,0 %, bezogen auf den Katalysatorformkörper, vorliegt.

3. Verfahren zur Herstellung eines Katalysatorformkörpers nach einem der Ansprüche 1 oder 2 umfassend ein Material der Formel CuAlₐMn_{b}Zn_{c}O_{d} und enthaltend Calciumaluminat als Bindermaterial in einem Gewichtsanteil von 0,5 bis 10,0 %, bezogen auf den Katalysatorformkörper, umfassend die folgenden Schritte:
a) Mischen eines metallhaltigen Gemischs, enthaltend Kupfer, Mangan und mindestens ein Element, ausgewählt aus Zink und Aluminium , mit Calciumaluminat, einem Schmiermittel und Wasser
b) Tablettierung der Mischung nach Schritt a) zum Erhalt eines tablettierten Formkörpers
c) Thermische Behandlung der tablettierten Formkörper bei einer Temperatur zwischen 200 und 800 °C für eine Dauer zwischen 30 min und 4 h,
wobei a zwischen 0 und 2,5, b zwischen 0,001 und 0,6 und c zwischen 0 und 2,5 beträgt, und d so gewählt ist, dass die Gesamtladung der Summenformel null ist.

4. Verfahren nach Anspruch 3, wobei das metallhaltige Gemisch ein Mischoxid von Kupfer, Mangan und Aluminium ist.

5. Verfahren nach einem der Ansprüche 3 oder 4, wobei das Calciumaluminat in einem Gewichtsanteil von 0,5 bis 5,0 %, bevorzugt von 0,5 % bis kleiner 5,0 %, noch bevorzugter 0,5 bis 3,0 %, bezogen auf den Katalysatorformkörper, vorliegt.

6. Verfahren nach einem der Ansprüche 3 bis 5, wobei die Mischung nach Schritt a) für eine Dauer von 5 min bis 10 h gealtert wird.

7. Verfahren nach einem der Ansprüche 3 bis 6, wobei die Thermische Behandlung zwischen 300 und 500 °C für eine Dauer zwischen 1 h und 3 h stattfindet.

8. Verfahren nach einem der Ansprüche 3 bis 7, wobei das in Schritt a) eingesetzte Calciumaluminat im Wesentlichen in oxidischer Form vorliegt.

9. Verfahren nach einem der Ansprüche 3 bis 8, wobei nach Schritt c) eine Reduzierung des Formkörpers erfolgt.

10. Verfahren zur Hydrierung von Carbonylgruppen in organischen Verbindungen mit dem Katalysatorformkörper nach einem der Ansprüche 1 oder 2 oder hergestellt nach einem Verfahren nach einem der Ansprüche 3 bis 9.

## Claims

1. Catalyst molding comprising a material of the formula CuAlₐMn_{b}Zn_{c}O_{d} , where a is a number between 0 and 2.5, b is a number between 0.001 and 0.6, c is a number between 0 and 2.5, and d is chosen such that the total charge of the molecular formula is zero, **characterized in that** the catalyst molded body is in tablet form and contains calcium aluminate as a binding material in a weight proportion of 0.5 to 10.0%, based on the catalyst molded body, and the lateral compressive strength is 100-300 N, determined in accordance with DIN EN 1094-5.

2. Catalyst molded body according to claim 1, wherein the calcium aluminate is present in a weight proportion of 0.5 to 5.0%, preferably from 0.5% to less than 5.0%, more preferably from 0.5 to 3.0%, based on the catalyst molded body.

3. Method for producing a catalyst molded body according to one of claims 1 or 2,comprising a material of the formula CuAlₐMn_{b}Zn_{c}O_{d}and containing calcium aluminate as a binder material in a weight proportion of 0.5 to 10.0%, based on the catalyst molded body, comprising the following steps:
a) mixing a metal-containing mixture containing copper, manganese, and at least one element selected from zinc and aluminum with calcium aluminate, a lubricant, and water
b) Tabletting the mixture according to step a) to obtain a tableted molded body
c) Thermal treatment of the tableted shaped bodies at a temperature between 200 and 800 °C for a duration between 30 min and 4 h,
where a is between 0 and 2.5, b is between 0.001 and 0.6, and c is between 0 and 2.5, and d is chosen such that the total charge of the sum formula is zero.

4. Method according to claim 3, wherein the metal-containing mixture is a mixed oxide of copper, manganese, and aluminum.

5. Method according to one of claims 3 or 4, wherein the calcium aluminate is present in a weight proportion of 0.5 to 5.0%, preferably from 0.5% to less than 5.0%, more preferably 0.5 to 3.0%, based on the catalyst molded body.

6. Method according to one of claims 3 to 5, wherein the mixture according to step a) is aged for a period of 5 min to 10 h.

7. Method according to one of claims 3 to 6, wherein the thermal treatment takes place between 300 and 500 °C for a duration of between 1 hour and 3 hours.

8. Method according to one of claims 3 to 7, wherein the calcium aluminate used in step a) is essentially present in oxide form.

9. Method according to one of claims 3 to 8, wherein after step c) a reduction of the molded body takes place.

10. Method for the hydrogenation of carbonyl groups in organic compounds using the catalyst molded body according to one of claims 1 or 2 or produced according to a method according to one of claims 3 to 9.

## Revendications

1. Catalyseur solide comprenant un matériau de formule CuAlₐMn_{b}Zn_{c}O_{d} , où a représente un nombre compris entre 0 et 2,5, b représente un nombre compris entre 0,001 et 0,6, c représente un nombre compris entre 0 et 2,5, et d est choisi de telle sorte que la charge totale de la formule brute soit nulle, **caractérisé en ce que** le corps moulé catalytique se présente sous forme de comprimés et contient de l'aluminate de calcium comme liant dans une proportion en poids de 0,5 à 10,0 % par rapport au corps moulé catalytique, et que la résistance à la compression latérale est de 100 à 300 N, déterminée selon la norme DIN EN 1094-5.

2. Catalyseur selon la revendication 1, dans lequel l'aluminate de calcium est présent dans une proportion en poids de 0,5 à 5,0 %, de préférence de 0,5 % à moins de 5,0 %, plus préféré de 0,5 à 3,0 %, par rapport au catalyseur.

3. Procédé de fabrication d'un corps moulé de catalyseur selon l'une des revendications 1 ou 2, comprenant un matériau de formule CuAlₐ Mn_{b} Zn_{c} O_{d} et contenant de l'aluminate de calcium comme liant dans une proportion en poids de 0,5 à 10,0 % par rapport au corps moulé de catalyseur, comprenant les étapes suivantes :
a) mélange d'un mélange contenant des métaux, comprenant du cuivre, du manganèse et au moins un élément choisi parmi le zinc et l'aluminium , avec de l'aluminate de calcium, un lubrifiant et de l'eau
b) comprimage du mélange selon l'étape a) pour obtenir un corps moulé comprimé
c) traitement thermique des corps moulés comprimés à une température comprise entre 200 et 800 °C pendant une durée comprise entre 30 minutes et 4 heures,
où a est compris entre 0 et 2,5, b est compris entre 0,001 et 0,6 et c est compris entre 0 et 2,5, et d est choisi de telle sorte que la charge totale de la formule brute soit nulle.

4. Procédé selon la revendication 3, dans lequel le mélange contenant des métaux est un oxyde mixte de cuivre, de manganèse et d'aluminium.

5. Procédé selon l'une des revendications 3 ou 4, dans lequel l'aluminate de calcium est présent dans une proportion en poids de 0,5 à 5,0 %, de préférence de 0,5 % à moins de 5,0 %, encore plus préféré de 0,5 à 3,0 %, par rapport au corps moulé du catalyseur.

6. Procédé selon l'une des revendications 3 à 5, dans lequel le mélange selon l'étape a) est vieilli pendant une durée de 5 minutes à 10 heures.

7. Procédé selon l'une des revendications 3 à 6, dans lequel le traitement thermique a lieu entre 300 et 500 °C pendant une durée comprise entre 1 h et 3 h.

8. Procédé selon l'une des revendications 3 à 7, dans lequel l'aluminate de calcium utilisé à l'étape a) se présente essentiellement sous forme oxydée.

9. Procédé selon l'une des revendications 3 à 8, dans lequel, après l'étape c), une réduction du corps moulé est effectuée.

10. Procédé d'hydrogénation de groupes carbonyles dans des composés organiques avec le corps moulé catalyseur selon l'une des revendications 1 ou 2 ou fabriqué selon un procédé selon l'une des revendications 3 à 9.
